# EUROPÄISCHE PATENTSCHRIFT

(11) **EP 1 596 778 B1**
(45) Veröffentlichungstag und Bekanntmachung des Hinweises auf die Patenterteilung: **17.12.2008**
(21) Anmeldenummer: 03775273.0
(22) Anmeldetag: 30.10.2003
(51) Int. Cl.: A61F 5/01, A61F 2/68, F15B 15/10

(54) **AKTIVE ORTHESE**
ACTIVE ORTHESIS
ORTHESE ACTIVE

(30) Priorität: 30.10.2002 DE 20216751 U; 30.10.2002 DE 20216749 U
(43) Veröffentlichungstag der Anmeldung: 23.11.2005
(73) Patentinhaber: Wassermann, Helmut, 81739 München (DE); Schostek, Sebastian, 80803 München (DE); Ho, Chi-Nghia, 72108 Rottenburg (DE)
(72) Erfinder: Wassermann, Helmut, 81739 München (DE); Schostek, Sebastian, 80803 München (DE); Ho, Chi-Nghia, 72108 Rottenburg (DE)
(74) Vertreter: Kunz, Herbert
(86) Internationale Anmeldenummer: PCT/EP2003/012123
(87) Internationale Veröffentlichungsnummer: WO 2004/039292

(56) Entgegenhaltungen:
- WO-A-01/13778
- DE-A- 3 918 955
- FR-A- 2 802 801
- GB-A- 2 260 495
- US-A- 4 557 257
- US-B1- 6 168 634
- DURFEE W K ET AL: "PRELIMINARY EVALUATION OF A CONTROLLED-BRAKE ORTHOSIS FOR REGULATING FES-AIDED GAIT" BIOMECHANICS, REHABILITATION, ELECTRICAL PHENOMENA, BIOMATERIALS. SAN DIEGO, OCT. 28 - 31, 1993, PROCEEDINGS OF THE ANNUAL INTERNATIONAL CONFERENCE OF THE ENGINEERING IN MEDICINE AND BIOLOGY SOCIETY, NEW YORK, IEEE, US, Bd. 3 CONF. 15, 28. Oktober 1993 (1993-10-28), Seiten 1234-1235, XP000452844

## Beschreibung

Die vorliegende Erfindung beschreibt eine aktive Körperhilfe für die Unterstützung der unteren Extremitäten des Trägers. Diese aktive Orthese (Powered Gait Orthosis, PGO) dient zur Unterstützung muskelschwacher Menschen durch einleiten zusätzlicher Kraftkomponenten in.den menschlichen Körper, die zu den vom Träger selbst aufgebrachten Kräften addiert werden. Die Erfindung wird angewendet zur Unterstützung aller Bewegungen, die ein Strecken des Knies und/oder des Hüftgelenks erfordern (Gehen, Treppensteigen, Aufstehen aus sitzender Position). Das Dokument FR-A-2802801 stellt den nächsten Stand der Technik dar.

Aktive Orthesen zur Unterstützung menschlicher Extremitäten werden heute vielfach eingesetzt zur Behandlung von Behinderungen der Arme und der Beine. Die derzeit verwendeten Systeme unterstützen vielfach gelähmte Extremitäten, weshalb sie in erster Linie stabilisierende Funktionen übernehmen. Bekannt ist beispielsweise ein System zur Unterstützung des menschlichen Bewegungsablaufes beim Gehen und verwendet dazu Gestänge und Gelenke, an die Aktuatoren adaptiert werden. Im Gegensatz dazu ist eine wesentliche Eigenschaft dieser Erfindung, dass zur Übertragung von Kräften und zur Einleitung dieser in den menschlichen Körper lediglich textile Strukturen zum Einsatz kommen.

Der menschliche Bewegungsapparat stellt viele technische Errungenschaften bei Bewegungs- und Transportsystemen in den Schatten. Es verwundert kaum, dass seit vielen Jahren vesucht wird, diesem Ideal der Lokomotion nahe zu kommen. Noch immer ist jedoch deutlich, dass die technischen Möglichkeiten zum Nachbau der menschlichen Biomechanik bei weitem nicht die Eigenschaften des natürlichen Vorbilds erreichen können. Allerdings stößt auch der menschliche Bewegungsapparat an seine Grenzen, wenn Komponenten nicht voll ausgebildet sind oder wenn in zunehmendem Alter oder bei Krankheiten Schwächen auftreten. Um Probleme dieser Art zu lösen-und um eine starke Einschränkung der Alltagsmobilität zu mindern, wird versucht, die Muskulatur durch Training zu stärken. Auch technische Lösungen, wie Rollstühle oder Treppenlifte, die die Fähigkeiten des natürlichen Bewegungsapparates ersetzen sollen, werden angeboten. Alternde und Menschen mit Muskelschwäche benötigen zudem oft pflegerische Unterstützung, um die einfachsten Tätigkeiten des Alltags, wie Essen oder Zähneputzen, selbständig bewältigen zu können. Technische Hilfen oder Pflege durch Dritte sind Lösungen, die von allen Beteiligten starke Kompromisse abverlangen. Zudem sind bisher eingesetzte technische Hilfssysteme, gleichgültig ob stationär oder mobil, mit hohen Kosten für das Gesundheitssystem verbunden.
Hier ist der Ansatzpunkt für die Entwicklung der adaptiven kraftverstärkenden Unterstützung des menschlichen Bewegungsapparates, der aktiven Orthese, die Gegenstand dieser Erfindung ist. Die unzureichenden körperlichen Fähigkeiten muskelschwacher Patienten sollen nicht mehr durch technische Alternativen ersetzt, sondern durch eine adaptive Mechanik unterstützt werden.

Die bisherigen Systeme von aktiven Orthesen (Powered Gait Orthosis, PGO) arbeiten mit Mechaniken aus starren Werkstoffen, um die verwendeten Kraftquellen (Elektromotoren, Pneumatik/Hydraulik, u.a.) integrieren zu können. Diese Konstruktionen erweisen sich als besonders unkomfortabel im Alltag, da einerseits die Komponenten viel Platz einnehmen, andererseits die verwendete Mechanik die Beweglichkeit des Körpers einschränkt und darüber hinaus eine erhebliche Peripherie erfordert, wodurch die Mobilität des Trägers erheblich eingeschränkt wird.
Die beschriebenen Probleme sollen mithilfe eines neuen Ansatzes bewältigt werden. Die hier vorgestellte aktive Orthese ist ein System verschiedener speziell angepasster Komponenten. Die Aktuatoren sind weich und flexibel, agieren ähnlich einem natürlichen Muskel und sind daher besonders für die Adaption an den menschlichen Bewegungsapparat geeignet. In Verbindung mit einer entsprechenden Sensorik können die Befehle zur Kontraktion von dem jeweils zu unterstützenden Muskel selbst kommen, dessen Aktionspotenzial gemessen wird. Die mit dem System verbundene Sensorik und Aktuatorik ermöglicht eine simultane Kraftverstärkung, und zwar über eine durch den Träger bestimmte willkürliche Kontrolle. Um den Anforderungen des Gesamtkonzeptes gerecht zu werden, wird die starre Mechanik bisheriger Systeme verworfen und durch eine textile Mechanik, in die die Aktuatorik und die Sensorik integriert werden, ersetzt. Der textile Aufbau dieser aktiven Orthese soll einen komfortablen Einsatz im Alltag ermöglichen. Da auch die integrierten Komponenten entsprechend klein, flach und flexibel gestaltet werden können, wird das Tragen der aktiven Orthese sogar unter alltäglicher Kleidung ermöglicht.

Die Konzeption der benötigten Sensorik und Steuerelektronik verwendet im Wesentlichen Technologien, die dem Stand der Technik entsprechen. Die Biosensorik ist für die Ermittlung des Unterstützungsbedarfs entscheidend. Um eine simultane Krafterzeugung zu ermöglichen, werden die Aktionspotenziale der zu unterstützenden Muskeln oder Muskelgruppen mithilfe eines EMGs gemessen. Die Steuerelektronik hat zum einen die Aufgabe, die Wechselspannungssignale der elektromyographischen Messungen in eine für die Aktuatorik verwertbare Form umzuwandeln, zum anderen muss sie die kraftverstärkende Wirkung der Aktuatorik sinnvoll einsetzen.

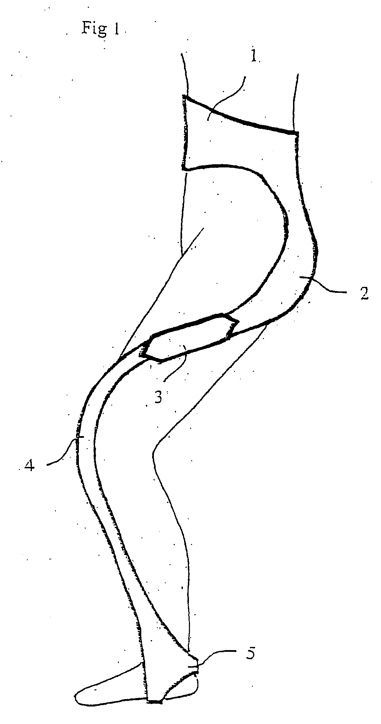

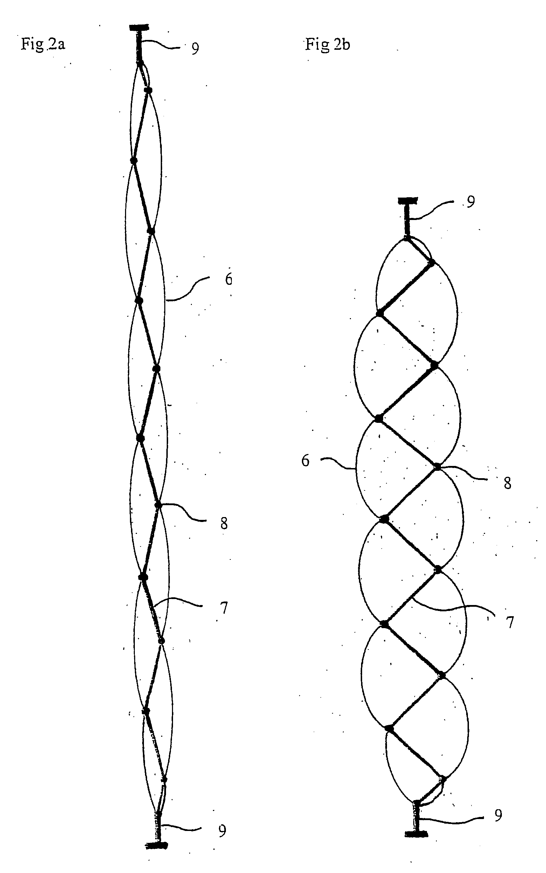

Als Aktuator kann ein neuartiges Gerät eingesetzt werden, das durch Kontraktion eine lineare Kraft erzeugt. Dieser technische Muskel ist ebenfalls Gegenstand der Erfindung. Lineare Kraftquellen übliche Bauart bedienen sich einer Spindel, Zahnstange oder Seilzug zur Umwandlung eines. Drehmomentes in eine Lineare Kraft oder pneumatischer bzw. hydraulischer Systeme. Bei Einsatz einer Spindel, Zahnstange oder Seilzuges ist ein gewisser Aufwand zur Integration des chemisch oder elektrisch betriebenen Aktuators in die anzutreibende Mechanik erforderlich. Auch Konstruktionen nach bionischem Ansatz (DE3918955A1) stützen sich auf eine verhältnismäßig steife Struktur. Ein biologischer Muskel dagegen ist flach und flexibel und damit auch an einen biologischen Bewegungsapparat adaptierbar. Zweck des hier vorgestellten Aktuators ist die von biologischen Muskeln bekannten positiven Eigenschaften in ein dem technischen Standard entsprechendes Gerät zu übersetzen, das an einen biologischen Bewegungsapparat adaptiert werden kann. Er verwendet eine flexible, pneumatisch oder hydraulisch betriebene Struktur zur Erzeugung einer linearen Kraft durch Kontraktion.
Diese Struktur besteht aus zugfesten, aber nicht steifen Außenwänden (6), die dicht gegenüber dem zur Druckerzeugung verwendeten Fluid sind. Diese Außenwände können zum Beispiel aus textilem Gewebe bestehen, das in ein Elastomer eingebettet ist. Zwischen den gegenüberliegenden Außenwänden verlaufen zugfeste Querverbindungen (7), die über Verbindungen (8) mit den Außenwänden verbunden sind. Diese Verbindungen können entweder zugfest sein oder das Verschieben der Querverbindungen gegenüber den Außenwänden zulassen. Die zugfesten Querverbindungen werden derart gestaltet, dass sie im entleerten Zustand parallel zur Kontraktionsrichtung verlaufen und bei Einbringen eines Fluids durch das Auseinanderstreben der Außenwände einen Winkel zur Kontraktionsachse bilden. Durch das Aufstellen der Querverbindungen kontrahiert die Struktur (Fig. 2a und 2b). An den beiden Enden der Struktur wird die durch Kontraktion erzeugte Kraft nach außen geführt. Diese Kraftanschlüsse (9) sind sowohl mit den Außenwänden als auch mit den Querverbindungen verbunden.
Durch den flachen, nicht steifen Aufbau eignet sich diese Struktur besonders zur Adaption an den menschlichen Bewegungspparat.

Durch Kontraktion der Aktuatoren wird mit dieser Erfindung die Streckung des Knie- und/oder des Hüftgelenks unterstützt: Dazu werde die Aktuatoren auf Höhe der Oberschenkel in eine textile Mechanik integriert. Diese textile Mechanik überträgt die durch die Aktuatorik erzeugten Kräfte und leitet diese in den Körper ein.
Die textile Mechanik der beinunterstützenden Orthese besteht aus zwei Teilen. Der obere Teil der textilien Mechanik setzt an der Taille an, wo er über einen Gürtel oder ein Korsett (1) mit dem Körper verbunden ist. Von dort läuft eine Stoffbahn dorsal über das Hüftgelenk (2), teilt sich und führt beidseitig am Oberschenkel nach vorne. Dort ist der obere Teile der textilen Mechanik mit der Aktuatorik (3) verbunden. Der untere Teil der textilen Mechanik (4) ist gegenüber dem oberen Teil der textilen Mechanik mit der Aktuatorik verbunden, verläuft ventral über das Kniegelenk und legt sich wie eine Schlaufe um die Ferse (5). Bei Kontraktion der Aktuatorik wird der Abstand zwischen dem oberen und dem unteren Teil der textilen Mechanik verkürzt und dadurch die Streckung des Beines unterstützt.

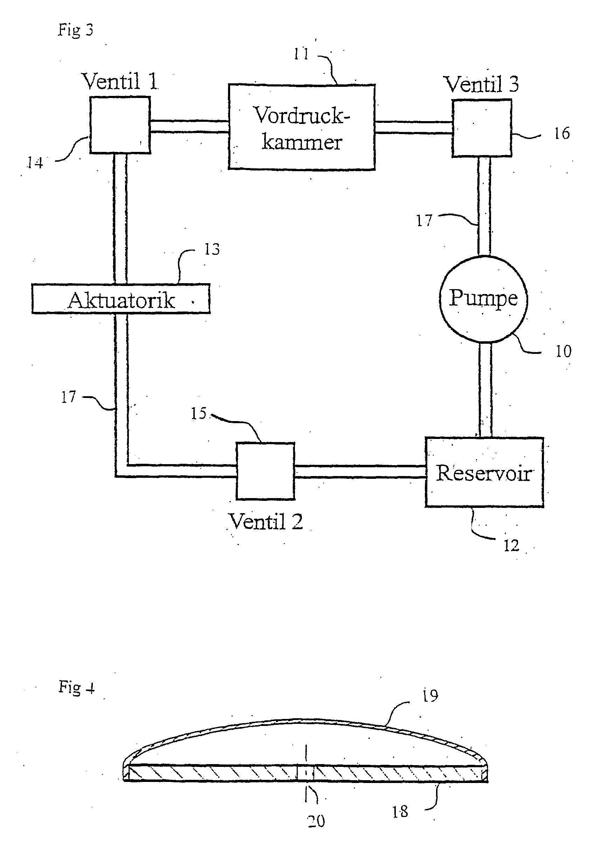

Die Aktuatorik kann hydraulisch betrieben werden. Die Druckerzeugung erfolgt mittels einem System mit Vordruckkammer. Diese besteht aus einer starren Grundplatte (18) mit mindestens einem hydraulischen Anschluss (20), über die eine elastische Membran (19) gespannt ist, siehe Fig. 4.

Eine Pumpe (10) pumpt das Fluid über hydraulische Leitungen (17) aus einem Fluid-Reservoir (12) in die Vordruckkammer (11), wodurch sich die elastische Membran nach außen wölbt. Wird eine Pumpe verwendet, die dem Rückfluss bei Stillstand nur einen geringen Widerstand entgegensetzt, muss zwischen Vordruckkammer und Fluid-Reservoir das Ventil 3 (16) eingesetzt werden. Der Druck in der Vordruckkammer kann durch Öffnen des Ventils 1 (14) auf die Aktuatorik (13) übertragen werden. Bei der Beugung des Knies wird das Ventil 2 (15) geöffnet und die Flüssigkeit zurück in das Fluid-reservoir gedrückt, siehe Fig. 3.

## Patentansprüche

1. Aktive Orthese mit einer kraftübertragenden Mechanik (1, 2, 4, 5) und einer Aktuatorik (3), **dadurch gekennzeichnet, dass**
die kraftübertragende Mechanik (1, 2, 4, 5) aus textilen Materialen besteht, die auf Zug belastbar sind, und
die Aktuatorik (3) auf Höhe der Oberschenkel eines Trägers der Orthese in die kraftübertragende Mechanik (1, 2, 4, 5) integriert ist und derart ausgebildet ist, dass sie eine Verkürzung der kraftübertragenden Mechanik und **dadurch** eine Streckung des Kniegelenks und/oder des Hüftgelenks beim Träger bewirken kann.

2. Aktive Orthese nach Anspruch 1, **dadurch gekennzeichnet, dass** die Aktuatorik (3) hydraulisch oder pneumatisch betrieben wird.

3. Aktive Orthese nach Anspruch 1 oder 2 zur Unterstützung der Beinbewegung, **dadurch gekennzeichnet, dass** der obere Teil (1, 2) der kraftübertragenden Mechanik über einen Gürtel oder ein Korsett oberhalb des Hüftgelenks mit dem menschlichen Körper kraftschlüssig verbunden ist, dorsal an dem Hüftgelenk nach unten verläuft, links und rechts des Oberschenkels des zu unterstützenden Beines nach unten verläuft und mit der Aktuatorik (3) nach Schutzanspruch 1 verbunden ist, und dass der untere Teil (4, 5) gegenüber dem oberen Teil mit der Aktuatorik (3) verbunden ist, ventral über das Kniegelenk verläuft und am Fuß mit dem menschlichen Körper kraftschlüssig verbunden ist.

4. Aktive Orthese nach einem der vorstehenden Ansprüche, **dadurch gekennzeichnet, dass** als Aktuatorik (3) pro Bein mindestens ein Aktuator vorgesehen ist.

5. Aktive Orthese nach einem der vorstehenden Ansprüche, bei der die Aktuatorik (3) als Aktuator eine Vorrichtung zur Erzeugung einer linearen Kraft durch Kontraktion der aktiven Orthese umfasst, **dadurch gekennzeichnet, dass** die Vorrichtung einen oder mehrere Hohlräume enthält, deren Volumen sich durch Einpumpen eines Fluids vergrößern lassen und **dadurch** eine lineare Kontraktion der Vorrichtung hervorrufen.

6. Aktive Orthese nach Anspruch 5, **dadurch gekennzeichnet, dass** der Aktuator zugfeste Außenwände (6) aus zwei parallelen Lagen eines Materials oder einer Struktur aufweist, die sich durch Kräfte mit Komponenten senkrecht zur Ebene der Lage verbiegen lassen, aber Kraftkomponenten parallel zur Ebene der Lage zugfest sind, und die dicht gegenüber dem verwendeten Fluid und an den Seiten miteinander undurchdringlich für das Fluid verbunden sind, und/oder **dadurch gekennzeichnet, dass** der Aktuator zugfeste Querverbindungen (7) aufweist, die eine bestimmte Länge haben und die Außenwände derart miteinander verbinden, dass sie bei entleertem Hohlraum parallel zur Kontraktionsrichtung der Vorrichtung angeordnet sind und bei Einpumpen eines Fluids in den Hohlraum durch das Auseinanderstreben der Außenwände mit einem bestimmten Winkel zur Kontraktionsrichtung der Vorrichtung angeordnet sind, und/oder **dadurch gekennzeichnet, dass** sie Kraftanschlüsse aufweist, die sich an den beiden Enden der Vorrichtung befinden und sowohl mit den zugfesten Außenwänden als auch mit den zugfesten Querverbindungen kraftschlüssig verbunden sind.

7. Aktive Orthese nach Anspruch 1, **dadurch gekennzeichnet, dass** zur Druckerzeugung eine elektrisch betriebene Pumpe (10) ein Fluid aus einem oder mehreren Reservoirs (12) in eine oder mehrere Vordruckkammern (11) pumpt, deren Druck durch Öffnen eines oder mehrerer Ventile (14) über fluidische Leitungen auf die Aktuatorik (13) übertragen werden kann, und dass durch Öffnen eines oder mehrerer anderer Ventile (15) das Fluid aus der Aktuatorik zurück in das Reservoir geleitet werden kann, oder **dadurch gekennzeichnet, dass** sie zur Druckerzeugung eine Vordruckkammer (12) aufweist, die eine starre Grundplatte (18) mit mindestens einem fluidischen Anschluss aufweist, über die eine elastische Membran (19) gespannt ist, die dicht gegenüber dem verwendeten Fluid mit der starren Grundplatte (18) verbunden ist.

8. Aktive Orthese nach Anspruch 1, **dadurch gekennzeichnet, dass** sie eine Steuerung mit einer Sensorik aufweist, die die Aktionen der Aktuatorik steuert.

9. Aktive Orthese nach Anspruch 8, **dadurch gekennzeichnet, dass** die Sensorik elektromyographische Messungen durchführen kann.

## Claims

1. An active leg brace with a load-transferring mechanism (1, 2, 4, 5) and an actuating element (3), **characterised in that**
the load-transferring mechanism (1, 2, 4, 5) is made of textile
materials which can be loaded by traction, and
the actuating element (3) is integrated into the load-transferring mechanism (1, 2, 4, 5) at the level of the thigh of a person wearing the leg brace and is designed such that it can bring about shortening of the load-transferring mechanism and thus bring about an extension of the knee joint and/or the hip joint of the wearer.

2. The active leg brace according to Claim 1, **characterised in that** the actuating element (3) is operated hydraulically or pneumatically.

3. The active leg brace according to Claim 1 or 2 for supporting the movement of the leg, **characterised in that** the upper part (1, 2) of the load-transferring mechanism is connected to the human body by friction fit by means of a belt or a corset above the hip joint, extends dorsally downwardly on the hip joint, extends downwardly on the left and on the right of the thigh of the leg to be supported, and is connected to the actuating element (3) according to protection Claim 1, and that the lower part (4, 5) is connected to the actuating element (3) in relation to the upper part, extends ventrally over the knee joint, and is connected by friction fit on the foot to the human body.

4. The active leg brace according to any one of the preceding claims, **characterised in that** at least one actuator per leg is provided as the actuating element (3).

5. The active leg brace according to any one of the preceding claims with which the actuating element (3) comprises as an actuator an apparatus for producing a linear force by contraction of the active leg brace, **characterised in that** the apparatus includes one or more cavities the volumes of which can be enlarged by pumping in a fluid, and thus bring about linear contraction of the apparatus.

6. The active leg brace according to Claim 5, **characterised in that** the actuator has outer walls (6) with tensile strength comprising two parallel layers of a material or a structure which can be bent by forces with components perpendicular to the level of the layer, but force components parallel to the level of the layer have tensile strength, and which are connected such as to form a seal against the fluid used, and to one another at the sides such as to be impenetrable to the fluid, and/or **characterised in that** the actuator has cross-connections (7) with tensile strength which are of a specific length and connect the outer walls to one another such that when the cavity is emptied they are disposed parallel to the direction of contraction of the apparatus, and when a fluid is pumped into the cavity, they are disposed at a specific angle to the direction of contraction of the apparatus due to the pulling apart of the outer walls, and/or **characterised in that** it has force attachments which are located at both ends of the apparatus and are connected by force fit both to the outer walls with tensile strength and to the cross-connections with tensile strength.

7. The active leg brace according to Claim 1, **characterised in that** in order to produce pressure an electrically operated pump (10) pumps a fluid out of one or more reservoirs (12) into one or more primary pressure chambers (11) the pressure of which can be transferred by opening one or more valves (14) by means of fluid lines to the actuating element (13), and that by opening one or more other valves (15) the fluid from the actuating element can be conveyed back into the reservoir, or **characterised in that** for producing pressure it has a primary pressure chamber (12) which has a rigid base plate (18) with at least one fluidic connection, wherein over the rigid base plate an elastic membrane (19) is stretched which is connected to the rigid base plate (18) such as to form a seal against the fluid used.

8. The active leg brace according to Claim 1, **characterised in that** it has a control with a sensor system which controls the actions of the actuating element.

9. The active leg brace according to Claim 8, **characterised in that** the sensor system can take electromyographic measurements.

## Revendications

1. Orthèse active comportant une mécanique transmettant les forces (1, 2, 4, 5) et un dispositif d'actionnement (3), **caractérisée en ce que**
la mécanique transmettant les forces (1, 2, 4, 5) se compose de matériaux textiles qui peuvent être soumis à des efforts de traction, et
le dispositif d'actionnement (3) est intégré, à hauteur de la cuisse du porteur de l'orthèse, dans la mécanique transmettant les forces (1, 2, 4, 5) et est conçu de manière à produire une contraction de la mécanique transmettant les forces et pour ainsi produire chez le porteur un étirement de l'articulation du genou et/ou de l'articulation de la hanche.

2. Orthèse active selon la revendication 1, **caractérisée en ce que** le dispositif d'actionnement (3) est entraîné par voie hydraulique ou pneumatique.

3. Orthèse active selon la revendication 1 ou 2 pour renforcer le mouvement de la jambe, **caractérisée en ce que** la partie supérieure (1, 2) de la mécanique transmettant les forces est reliée au corps humain par liaison de force grâce à une ceinture ou à un corset au-dessus de l'articulation de la hanche, en se prolongeant dorsalement vers le bas au niveau de l'articulation de la hanche, en continuant vers le bas à gauche et à droite de la cuisse de la jambe à renforcer, en étant reliée au dispositif d'actionnement (3) selon la revendication 1, et **en ce que** la partie inférieure (4, 5) est reliée en regard de la partie supérieure avec le dispositif d'actionnement (3), en se prolongeant ventralement sur l'articulation du genou et en étant reliée au corps humain au niveau du pied par liaison de force.

4. Orthèse active selon l'une des revendications précédentes, **caractérisée en ce qu'**on prévoit comme dispositif d'actionnement (3) au moins un actionneur par jambe.

5. Orthèse active selon l'une des revendications précédentes, dans laquelle le dispositif d'actionnement (3) comprend, comme actionneur, un dispositif permettant de produire une force linéaire par contraction de l'orthèse active, **caractérisée en ce que** le dispositif contient une ou plusieurs cavités dont les volumes peuvent être accrus par pompage d'un fluide pour provoquer une contraction linéaire du dispositif.

6. Orthèse active selon la revendication 5, **caractérisée en ce que** l'actionneur présente des parois extérieures (6) résistantes à la traction qui sont formées de deux couches parallèles de matériau ou de structure, qui sont susceptibles de fléchir sous l'effet de forces avec des composantes perpendiculairement au plan de la couche, mais des composantes de force qui sont résistantes à la traction parallèlement au plan de la couche, et qui sont reliées de façon étanche par rapport au fluide employé en étant reliées sur les côtés de façon imperméable vis-à-vis du fluide, et/ou **caractérisée en ce que** l'actionneur présente des liaisons transversales (7) résistantes à la traction qui ont une longueur spécifique et relient les parois extérieures entre elles de manière à se placer parallèlement à la direction de contraction du dispositif lorsque la cavité est vide et à se placer avec un certain angle par rapport à la direction de contraction du dispositif lors du pompage d'un fluide dans la cavité, sous l'effet de l'écartement des parois extérieures, et/ou **caractérisée en ce qu'**elle présente des raccords de force qui se trouvent aux deux extrémités du dispositif et qui sont reliés par liaison de force aux parois extérieures résistantes à la traction ainsi qu'aux liaisons transversales résistantes à la traction.

7. Orthèse active selon la revendication 1, **caractérisée en ce qu'**une pompe électrique (10) pompe un fluide provenant d'un ou de plusieurs réservoirs (12) dans une ou plusieurs chambres de précompression (11) afin de produire une pression qui peut être transmise au dispositif d'actionnement (13) par des lignes fluidiques grâce à l'ouverture d'une ou de plusieurs soupapes (14) et **en ce que** l'ouverture d'une ou de plusieurs autres soupapes (15) permet de renvoyer dans le réservoir le fluide présent dans le dispositif d'actionnement, ou **caractérisée en ce qu'**elle présente, pour produire une pression, une chambre de précompression (12) présentant une plaque de base (18) fixe comportant au moins un raccord fluidique sur lequel est tendue une membrane élastique (19) reliée à la plaque de base (18) de façon étanche par rapport au fluide employé.

8. Orthèse active selon la revendication 1, **caractérisée en ce qu'**elle présente un dispositif de commande pourvu d'une technique sensorielle pour commander les actions du dispositif d'actionnement.

9. Orthèse active selon la revendication 8, **caractérisée en ce que** la technique sensorielle est capable d'effectuer des mesures électromyographiquess.
